# EUROPÄISCHE PATENTSCHRIFT

(11) **EP 0 546 331 B1**
(45) Veröffentlichungstag und Bekanntmachung des Hinweises auf die Patenterteilung: **05.02.1997**
(21) Anmeldenummer: 92119165.6
(22) Anmeldetag: 09.11.1992
(51) Int. Cl.: A61F 5/01

(54) **Einstellbares Orthesengelenk**
Adjustable motion brace
Orthèse d'articulation ajustable

(30) Priorität: 11.11.1991 DE 4137057
(43) Veröffentlichungstag der Anmeldung: 16.06.1993
(73) Patentinhaber: BIEDERMANN MOTECH GmbH, D-78054 Villingen-Schwenningen (DE)
(72) Erfinder: Biedermann, Lutz, c/o Biedermann Motech GmbH, W-7730 VS-Schwenningen (DE)
(74) Vertreter: Prüfer, Lutz H., Dipl.-Phys.

(56) Entgegenhaltungen:
- EP-A- 0 454 186
- EP-A- 0 546 330
- GB-A- 2 215 394
- US-A- 4 928 676

## Beschreibung

Die Erfindung betrifft ein Orthesengelenk nach dem Oberbegriff des Patentanspruches 1.

Ein derartiges Orthesengelenk ist aus der US-PS 4.886.054 bekannt. Zur Begrenzung der Streckbewegung bzw. der Beugebewegung des Gelenkes ist ein auf der Lagerplatte montiertes Führungsgehäuse vorgesehen, welches einen Schlitz aufweist. Es ist mit dem oberen Anschlußteil ein Hebel verbunden, der an seinem freien Ende eine Kugel aufweist. Der Hebel ist so in das Gehäuse eingesetzt, daß die Kugel im inneren verläuft und der Hebel sich durch den Schlitz nach außen erstreckt. Die Länge der Führung im Gehäuse ist durch zwei an den gegenüberliegenden Enden vorgesehenen Schrauben, die jeweils Anschläge bilden, begrenzt. Diese Konstruktion der Anschlagsbegrenzung ist außerordentlich kompliziert und aufwendig. Darüber hinaus weist sie den Nachteil auf, daß sie wegen der teilweise schräg angreifenden Kräfte auch zu Funktionsführungen führt. Ferner resultiert die Tatsache, daß die Kugel jeweils an den Anschlägen direkt anschlägt, zu einer unangenehmen Trageigenschaft beim jeweiligen Anschlag.

Die US-PS 4,928,676 zeigt ebenfalls ein Orthesengelenk mit den Merkmalen des Oberbegriffes des Patentanspruches 1. Es ist dort eine Dämpfung eines in Extension gesperrten Kniegelenkes vorgesehen. Dieses Orthesengelenk ist nicht zur therapeutischen Behandlung geeignet.

Aufgabe der Erfindung ist es, ein Orthesengelenk nach dem Oberbegriff des Anspruches 1 zu schaffen, welches einerseits angenehme Trageigenschaften besitzt, andererseits aber auch eine vereinfachte Einstelleinrichtung aufweist.

Diese Aufgabe wird durch das im Patentanspruch 1 gekennzeichnete einstellbare Orthesengelenk gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:
- Fig. 1: einen Schnitt entlang der Linie A-A in Fig. 3, in gestreckter Endstellung des Orthesengelenkes;
- Fig. 2: dieselbe Gelenkdarstellung in gebeugter Endstellung;
- Fig. 3: eine Draufsicht auf das Orthesengelenk von vorn;
- Fig. 4: das in Fig. 2 gezeigte Orthesengelenk von der Rückseite her mit gestrichelt angedeuteter Pelotte;
- Fig. 5: das in Fig. 1 gezeigte Orthesengelenk von der Rückseite her mit gestrichelt angedeuteter Pelotte;
- Fig. 6: ein Detail aus Fig. 1 in vergrößerter Darstellung; und
- Fig. 7: ein weiteres Detail aus Fig. 1 in vergrößerter Darstellung.

Das Orthesengelenk weist eine erste Lagerplatte (1) auf. Mit dieser ist ein Oberschenkel-Anschlußteil (2) über eine sich senkrecht zur Ebene der Lagerplatte erstreckende Lagerbuchse (3) schwenkbar verbunden. Ferner ist ein Unterschenkel-Anschlußteil (4) vorgesehen, welches über eine sich senkrecht zur Ebene der Lagerplatte erstreckende zweite Lagerbuchse (5) mit der Lagerplatte (1) schwenkbar verbunden ist. Ein Verbindungsglied (6) ist zwischen den beiden Anschlußteilen vorgesehen. Dieses ist an seinem einen Ende über eine dritte Lagerbuchse (7) schwenkbar mit dem Unterschenkel-Anschlußteil (4) und an seinem anderen Ende über eine vierte Lagerbuchse (8) mit dem Oberschenkel-Anschlußteil (2) schwenkbar verbunden.

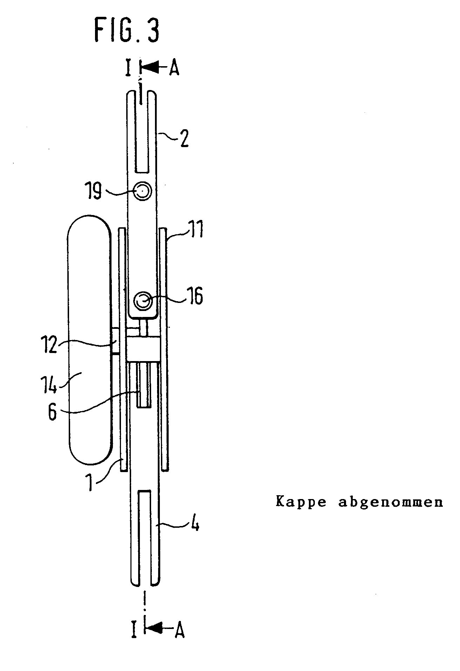

Die Verbindung zwischen dem Verbindungsglied und den Anschlußteilen ist so hergestellt, daß die Anschlußteile an ihren einander zugewandten Enden sich parallel zur Lagerplatte erstreckende schlitzförmige Ausnehmungen (9, 10) aufweisen. In diesen ist das Verbindungsglied bewegbar geführt. Wie am besten aus Fig. 3 ersichtlich ist, ist eine zweite Lagerplatte (11) vorgesehen, die insoweit zur ersten Lagerplatte spiegelsymmetrisch ausgebildet ist. Die jeweiligen Lagerbuchsen sind mit ihrem der ersten Lagerplatte abgewandten Ende in der zweiten Lagerplatte gelagert. Die Anschlußteile können sich zwischen den beiden Lagerplatten frei bewegen.

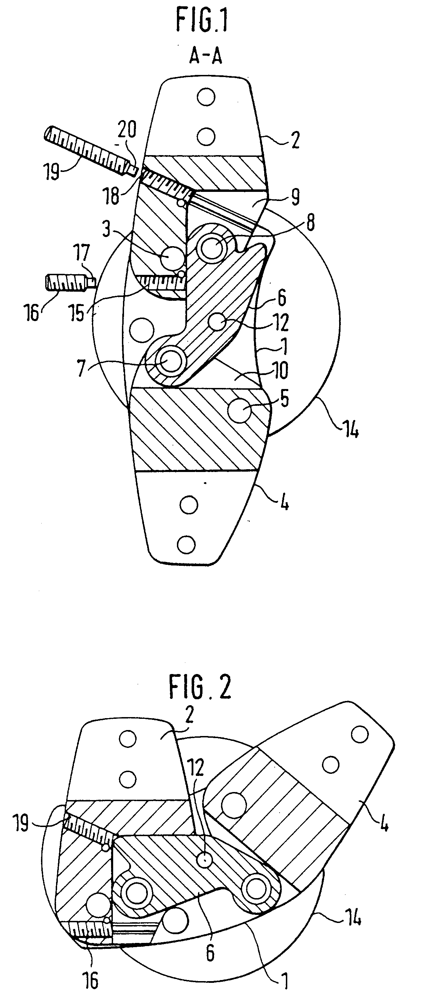

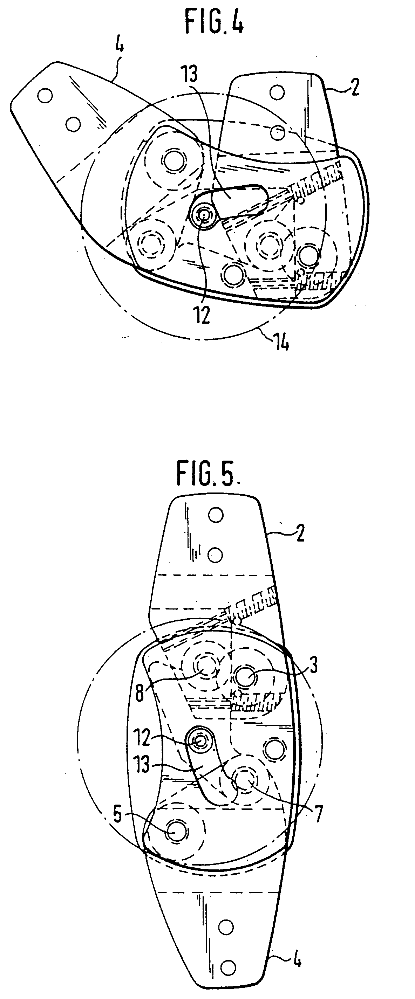

Die Lage der ersten bis vierten Lagerbuchsen ist in an sich bekannnten Weise so gewählt, daß die die erste und zweite Lagerbuchse verbindende erste Gerade und die die dritte und vierte Lagerbuchse verbindende zweite Gerade sich unter einem Winkel derart schneiden, daß sich bei der Bewegung der Anschlußteile bzw. der damit verbundenen Orthese aufgrund der zwei durch die erste und zweite Lagerbuchse gebildeten Gelenke eine Bewegung mit einem annähernd kreisförmigen Abschnitt und einem sich daran anschließenden annähernd schneckenförmigen Abschnitt ergibt. Das Verbindungsglied (6) weist eine Gewindebuchse (12) auf. In den Figuren 1 und 2 ist die Rückseite des die Gewindebuchse mit dem Verbindungsglied verbindenden Niets erkennbar, während die Gewindebuchse selbst in den Figuren 3 bis 5 zu sehen ist. Die erste Lagerplatte (1) weist eine Ausnehmung (13) auf, die so groß ist, daß die Gewindebuchse (12) sich bei der Bewegung des Orthesengelenkes frei durch die Ausnehmung hindurch bewegen kann. Eine Pelotte (14) ist mittels einer geeigneten Schraube über die Gewindebuchse (12) fest mit dem Verbindungsglied (6) verbunden. Die Gewindebuchse (12) ist, wie am besten aus Fig. 1 ersichtlich ist, auf einer Linie angeordnet, die sich senkrecht zur Längsachse des gestreckten Orthesengelenkes erstreckt und die einen etwa gleichen Abstand von der ersten Lagerbuchse (3) und von der zweiten Lagerbuchse (5) aufweist. Seitlich gesehen liegt die Stelle auf einer Geraden, die sich parallel zur Längsachse des gestreckten Orthesengelenkes erstreckt und etwa den gleichen Abstand von der ersten und von der zweiten Lagerbuchse aufweist. Durch die feste Verbindung der Pelotte mit dem Verbindungsglied (6) wird erreicht, daß diese sich bei der Bewegung des Orthesengelenkes nicht mehr auf und ab bzw. hin und her bewegt.

Wie am besten aus den Figuren 1 und 2 ersichtlich ist, ist in der Mittenebene des Oberschenkel-Anschlußteiles (2) eine erste sich parallel zur Lagerplatte erstreckende Gewindebohrung (15) vorgesehen. In dieser ist eine Gewindehülse (16), die einen durch eine innere Feder vorgespannten Anschlag (17) aufweist, eingeschraubt. Die Schraube ist in Abhängigkeit von den Bedürfnissen des Patienten so eingestellt, daß die in Fig. 1 gezeigte gestreckte Endstellung durch den Anschlag begrenzt wird. Ferner ist eine zweite Gewindebohrung (18) vorgesehen, in die eine entsprechende Gewindehülse (19) mit einem durch eine innnere Feder vorgespannten Anschlag (20) eingeschraubt ist. Durch die eingeschraubte Stellung der Gewindehülse mit dem Anschlag wird, wie insbesondere aus Fig. 2 ersichtlich ist, die gebeugte Endstellung des Orthesengelenkes begrenzt. Durch die Federvorspannung bzw. gewünschtenfalls auch eine durch einen Gummi erzeugte elastische Vorspannung wird das harte Anschlagen der Orthesenteile beim Tragen verhindert, so daß der Tragkomfort erheblich verbessert wird.

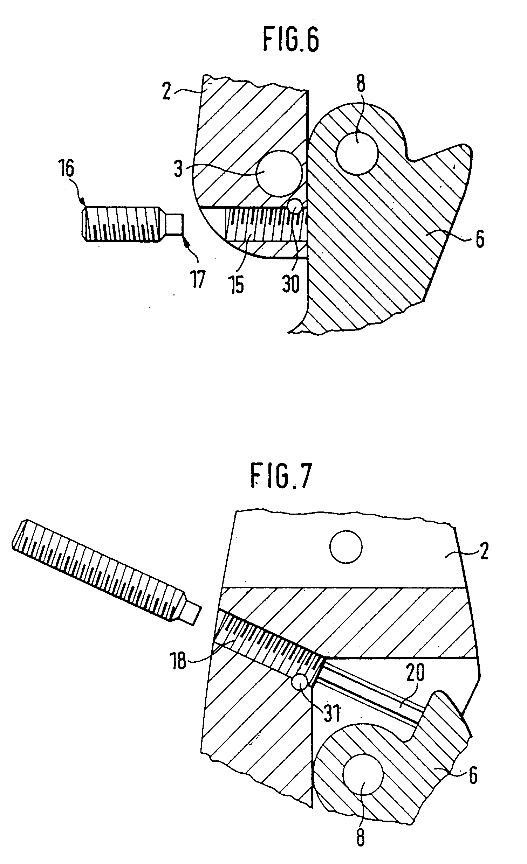

In den Figuren 6 und 7 sind vergrößerte Detaildarstellungen für die abgepufferten Anschläge gezeigt. In Figur 6 ist das Wiederlager im ausgeschraubten Zustand gezeigt, in Figur 7 ist ein Anschlag im eingeschraubten Zustand, ein weiterer zur Demonstration im ausgeschraubten Zustand gezeigt. Wie insbesondere aus diesen beiden Figuren ersichtlich ist, ist jeweils eine asymmetrisch gelagter Feststellstift (30) bzw. (31) so vorgesehen, daß durch Verdrehen dieses Feststellstiftes in die Arretierstellung ein versehentliches Verstellen der eingesetzten Schraube mit dem wie oben beschrieben federnd ausgebildeten Wiederlager unmöglich ist.

In dem oben beschriebenen Ausführungsbeispiel sind die beiden die Anschläge bildenden Schraubenhülsen in dem Oberschenkel-Anschlußteil vorgesehen. Grundsätzlich ist es auch möglich, das Unterschenkel-Anschlußteil anders zu formen und die Anschläge im Unterschenkel-Anschlußteil vorzusehen. Ferner ist es möglich, einen der Anschläge in einem der Anschlußteile und den anderen im anderen der Anschlußteile anzubringen.

## Patentansprüche

1. Orthesengelenk mit einer ersten Lagerplatte (1), einem mit dieser über eine erste Lagerbuchse (3) schwenkbar verbundenen Oberschenkel-Anschlußteil (2),
einem mit der ersten Lagerplatte (1) über eine zweite Lagerbuchse (5) schwenkbar verbundenen Unterschenkel-Anschlußteil (4),
einem mit den beiden Anschlußteilen (2, 4) über eine dritte und eine vierte Lagerbuchse (7, 8) schwenkbar verbundenen Verbindungsglied (6),
dadurch gekennzeichnet, daß eines der Anschlußteile (2) und das Verbindungsglied (6) direkt über wenigstens einen ersten Anschlag (17, 20) miteinander in Eingriff kommen und dieser Anschlag einstellbar ist.

2. Orthesengelenk nach Anspruch 1, dadurch gekennzeichnet, daß wenigstens einer der Anschläge elastisch ausgebildet ist.

3. Orthesengelenk nach Anspruch 1 oder 2, dadurch gekennzeichnet, daß die Anschläge als in einer Gewindebohrung (15, 18) gehaltene Schraubhülsen (16, 19) mit federvorgespannten Anschlagköpfen (17, 20) ausgebildet sind.

4. Orthesengelenk nach einem der Ansprüche 1 bis 3, dadurch gekennzeichnet, daß die Anschläge (17, 20) in einem der Anschlußteile (2) vorgesehen sind und sich in eine schlitzförmige Ausnehmung (9) in dem Anschlußteil (2) hinein erstrecken.

5. Orthesengelenk nach einem der Ansprüche 1 bis 4, dadurch gekennzeichnet, daß eine mit dem Verbindungsglied (6) fest verbundene Pelotte (14) vorgesehen ist.

6. Orthesengelenk nach Anspruch 5, dadurch gekennzeichnet, daß die Mitte der Pelotte (14) im wesentlichen auf einer Linie liegt, die senkrecht zur Längsrichtung des gestreckten Orthesengelenkes verläuft und annähernd den gleichen Abstand von der ersten und zweiten Lagerbuchse (3, 5) aufweist.

7. Orthesengelenk nach einem der Ansprüche 1-6 dadurch gekennzeichnet, daß das eine der Anschlußteile (2) und das Verbindungsglied (6) direkt über einen ersten Anschlag und das andere Anschlußteil und das Verbindungsglied direkt über einen zweiten Anschlag miteinander in Eingriff kommen und diese Anschläge einstellbar sind.

8. Orthesengelenk nach einem der Ansprüche 5 bis 7, dadurch gekennzeichnet, daß eine zweite Lagerplatte (11) vorgesehen ist und die Lagerbuchsen (3, 5) in den beiden Lagerplatten (1, 11) gelagert sind, und eine der Lagerplatten eine Ausnehmung (13) aufweist, durch die sich die Verbindung zwischen dem Verbindungsglied (6) und der Pelotte (14) erstreckt.

## Claims

1. Orthesis joint with a first bearing plate (1), a femoral attachment part (2) which is connected pivotably to the first bearing plate (1) via a first bearing bushing (3), a tibial attachment part (4) which is connected pivotably to the first bearing plate (1) via a second bearing bushing (5), a connection member (6) which is connected pivotably to the two attachment parts (2, 4) via a third and a fourth bearing bushing (7, 8), characterized in that one of the attachment parts (2) and the connection member (6) engage with one another directly via at least a first limit stop (17, 20), and this limit stop is adjustable.

2. Orthesis joint according to Claim 1, characterized in that at least one of the limit stops is designed elastically.

3. Orthesis joint according to Claim 1 or 2, characterized in that the limit stops are designed as screw sleeves (16, 19) which are held in a threaded bore (15, 18) and which have limit stop heads (17, 20) pretensioned by spring means.

4. Orthesis joint according to one of Claims 1 to 3, characterized in that the limit stops (17, 20) are provided in one of the attachment parts (2) and extend into a slot-shaped recess (9) in the attachment part (2).

5. Orthesis joint according to one of Claims 1 to 4, characterized in that a pad (14) is provided which is firmly connected to the connection member (6).

6. Orthesis joint according to Claim 5, characterized in that the middle of the pad (14) lies essentially on a line which runs perpendicular to the longitudinal direction of the extended orthesis joint and is at approximately the same distance from the first and the second bearing bushing (3, 5).

7. Orthesis joint according to one of Claims 1 - 6, characterized in that one of the attachment parts (2) and the connection member (6) engage with one another directly via a first limit stop, and the other attachment part and the connection member engage with one another directly via a second limit stop, and these limit stops are adjustable.

8. Orthesis joint according to one of Claims 5 to 7, characterized in that a second bearing plate (11) is provided and the bearing bushings (3, 5) are mounted in the two bearing plates (1, 11), and one of the bearing plates has a recess (13) through which the connection between the connection member (6) and the pad (14) extends.

## Revendications

1. Orthèse d'articulation comprenant une première plaque support (1), une pièce de raccordement de cuisse (2) reliée de façon pivotante à cette plaque par un premier coussinet (3), une pièce de raccordement de jambe (4) reliée de façon pivotante à la première plaque support (1) par un deuxième coussinet (5), un élément de liaison (6) relié de façon pivotante aux deux pièces de raccordement (2, 4) par un troisième et un quatrième coussinets (7, 8), caractérisé en ce que l'une des pièces de raccordement (2) et l'élément de liaison (6) s'emboîtent l'un dans l'autre directement au moyen d'au moins une première butée (17,20) et cette butée est réglable.

2. Orthèse d'articulation selon la revendication 1, caractérisée en ce qu'au moins l'une des butées est conçue de façon élastique.

3. Orthèse d'articulation selon la revendication 1 ou 2, caractérisée en ce que les butées se présentent sous la forme de douilles à vis (16, 19) maintenues dans un alésage fileté (15, 18) et sont pourvues de têtes de butée (17, 20) chargées par ressort.

4. Orthèse d'articulation selon l'une quelconque des revendications 2 à 3, caractérisée en ce que les butées (17, 20) sont prévues dans l'une des pièces de raccordement (2) et s'étendent dans un évidement (9) en forme de fente dans la pièce de raccordement (2).

5. Orthèse d'articulation selon l'une quelconque des revendications 1 à 4, caractérisée en ce qu'il est prévu une pelote (14) reliée de façon fixe à l'élément de liaison (6).

6. Orthèse d'articulation selon la revendication 5, caractérisée en ce que le centre de la pelote (14) se situe sensiblement sur une ligne qui est perpendiculaire à la direction longitudinale de l'orthèse d'articulation étirée et présente approximativement la même distance par rapport au premier et au deuxième coussinets (3, 5).

7. Orthèse d'articulation selon l'une quelconque des revendications 1 à 6, caractérisée en ce que l'une des pièces de raccordement (2) et l'élément de liaison (6) s'emboîtent directement par une première butée et en ce que l'autre pièce de raccordement et l'élément de liaison s'emboîtent directement par une deuxième butée et ces butées sont réglables.

8. Orthèse d'articulation selon l'une quelconque des revendications 5 à 7, caractérisée en ce qu'une deuxième plaque support (11) est prévue et les coussinets (3, 5) sont logés dans les deux plaques supports (1, 11) et l'une des plaques supports présente un évidement (13) par lequel la liaison s'étend entre l'élément de liaison (6) et la pelote (14).
